# EUROPEAN PATENT APPLICATION

(11) **EP 3 763 416 A1**
(43) Date of publication of application: **13.01.2021**
(21) Application number: 20170052.3
(22) Date of filing: 17.04.2020
(51) Int. Cl.: A61N 1/37, A61N 1/375, A61N 1/36

(54) **DIELECTRIC HEADER STRUCTURE FOR REDUCING ELECTROMAGNETIC INTERFERENCE**

(30) Priority: 11.07.2019 US 201962872718 P
(71) Applicant: BIOTRONIK SE & Co. KG, 12359 Berlin (DE)
(72) Inventor: KREFT, Isaac, Hillsboro, OR 97123 (US); BROWN, James E., Tigard, OR 97224 (US)
(74) Representative: Heinz, Benjamin

(57) **Abstract**

The invention relates to an implantable medical device (1), comprising: a header (2) attached to a housing (5) of the device (1), and at least one electrode (7) which is electrically conductively connected or connectable to the header (2), the at least one electrode (7) comprising at least one electrode contact (73a) for contacting tissue of the patient and for applying electrical stimulation pulses to the tissue and/or detecting electrical pulses from the tissue. According to the invention, the header (2) comprises a dielectric material (3) that is configured to capacitively couple RF energy acting on the device (1) to a conductor (5, 7, 50) of the implantable medical device (1) or to the tissue of the patient when the tissue touches the header (2) of the device (1).

## Description

Today, after implantation, medical implants are exposed to electromagnetic fields from a variety of sources, including high-performance radios, wireless energy storage charging, electronic article surveillance, etc. The high (e.g. radio) frequency (RF) and gradient fields of an MRI scanner pose a particular risk to the patient when the patient is examined by the MRI scanner. These fields can induce electrical currents in a lead of the implant, especially in the electrode lead of an active cardiac or neurostimulation implant, which flow into the implant itself. This unwanted RF-induced electrical current can, for example in the case of an implant for the treatment of cardiac arrhythmias, lead to unintentional stimulation or to the detection of false heart signals and even to a malfunction of the device, which in the worst case can lead to arrhythmias and even death of the patient. The currents can also be reflected at the implant and cause MRI RF-induced tissue heating near the lead electrode at the distal end.

In order to suppress the effects of such fields, feedthroughs equipped with filters for electrical conductors are known as well as discrete components for EMI filtering in the respective module or implant. However, the additional filters in the electrical feedthrough or implant (EMI) require additional components, which increase the complexity of the design.

Alternatively, measures to protect against high-frequency electromagnetic fields in an implant could be dispensed with in order not to increase the complexity of the design of implantable medical devices. However, waiving the EMI filtering capability of the medical device is generally not a desirable solution to the problem, as patients with implants that are not MRI-conditional will not be able to use this increasingly widespread technology.

Based on the above, the invention is therefore based on the problem of diverting the energy transported by high-frequency (RF) fields from a header of the implant or from the implant without significantly changing the stimulation parameters of the implant, and particularly to mitigate RF-induced malfunction and unintended stimulation.

This problem is solved by an implantable medical device (also denoted as implant) having the features of claim 1. Advantageous embodiments of the invention are described below and are stated in the sub claims.

According to claim 1, an implantable medical device is disclosed comprising:
- a header portion (also referred to as header) and attached to a housing of the device, the housing preferably containing an energy source of the device and electronic components of the device, in particular a pulse generator for generating electrical stimulation pulses and/or an electronic component for sensing electric pulses, e.g. from a tissue, and
- at least one electrode which is electrically connected or can be connected to the header, or which at least one electrode is arranged on or extends through the header portion, wherein the at least one electrode comprises at least one electrode contact for contacting tissue of the patient and for delivering electrical stimulation pulses into the tissue and/or for detecting electrical pulses.

According to the invention, it is now provided that a dielectric material is arranged in the header which is configured to capacitively couple RF energy acting on the device into the tissue of the patient when this tissue contacts the header of the device. Alternatively, the RF energy can be coupled into adjacent channels or a conductor connected to the housing of the device.

The implantable medical device of the invention may comprise an electrode lead which comprises the above described at least one electrode. The electrode lead comprises a distal end configured to electrically contact a tissue for delivering or sensing electrical pulses and a proximal end configured to electrically connect the electrode lead with the pulse generator and/or electronic components of the implantable medical device. Preferably, the proximal end of the electrode lead is received by a receptacle within the header of the implantable medical device, which is particularly formed by a contact assembly, e.g. a contact socket. Here, the contact assembly is configured to electrically contact the proximal end of the lead and is electrically connected to the pulse generator and/or electronic components of the implantable medical device within the housing of the implantable medical device. The contact assembly may be electrically connected to the pulse generator and/or electronic components of the implantable medical device within the housing via an electric feedthrough, wherein particularly the contact assembly may be electrically connected to one or more pins of the electric feedthrough by an electric conductor, respectively, e.g. a metal ribbon. Furthermore, the implantable medical device of the invention may be designed as pacemaker, cardioverter/defibrillator or neurostimulator.

Alternatively, the implantable medical device may be designed as an intracardiac pacemaker, wherein the at least one electrode is connected to a housing of the leadless pacemaker and forms an end section of the leadless pacemaker. Here, the electrode is arranged on or extends through the header and is not connected to the latter as an external elongated electrode lead. Therefore, such intracardiac pacemakers are commonly termed as leadless pacemakers.

The implantable medical device may also be designed as a loop recorder, wherein the electrode is designed in the form of an elongated electrode, which is arranged at one end of the loop recorder and extends through the header of the loop recorder.

In case the implantable medical device is designed as an intracardiac pacemaker and the electrode is arranged on the header portion, the electrode is preferably electrically connected to the electronic circuitry in an internal space of the housing of the leadless pacemaker via an electrically conducting pin that particularly extends through the header (see also below). Alternatively in case the electrode extends through the header portion, the electrode is formed by the above mentioned electrically conductive pin connected to the electronic circuitry in the internal space of the housing.

Particularly, a dielectric material is arranged in the header and is configured to capacitively couple RF energy acting on the device to a conductor of the implantable medical device or to the tissue of the patient when the tissue touches the header of the device, wherein in an embodiment, said conductor is the housing of the implantable medical device. Preferably, the housing is formed out of a metal or comprises a metal, wherein the metal can be titanium.

According to an embodiment of the invention, the electrode is an electrode lead, preferably an elongated flexible electrode lead, and wherein preferably the at least one electrode contact is arranged on an outside of the electrode lead. The at least one electrode contact can be an annular electrode contact.

Furthermore, according to an embodiment of the present invention, the dielectric material is crystalline barium titanate or titanium dioxide.

Particularly, in an embodiment, the dielectric material is arranged between two electrically conductive structures of the header portion.

According to a further embodiment, the dielectric material is comprised by at least one of: an epoxy coating of the header, a preformed polysulfone section of the header, a contact seal of the header, a spacer of the header, an adjusting screw seal of the header

According to a further embodiment, the header portion comprises a capacitor containing the dielectric material. Particularly, the header portion can comprise a conductor structure that is widened to form a plate of the capacitor.

According to a preferred embodiment, the implantable medical device is an implantable leadless pacemaker, i.e. an intracardiac pacemaker, that is configured to apply electrical pacing pulses to a heart of a patient. Such a leadless pacemaker does not comprise an electrode lead but an electrode having an electrode contact that is formed on the header of the implantable medical device.

According to an embodiment, the header portion of the implantable medical device (particularly implantable leadless pacemaker) comprises a circumferential header cap and a header insert arranged in a through opening of the header cap. Particularly, also the header insert comprises a through opening through which an electrically conducting pin extends that is electrically conductively connected to the electrode. Preferably, the pin is connected to a mounting plate of the electrode, wherein the pin extends through an electrically insulating body that is connected to a circumferential metallic flange of the housing. Alternatively, the electrically conductive pin forms the electrode of the implantable medical device.

According to a further embodiment, the implantable medical device comprises a plurality of tines for anchoring the implantable medical device to tissue, particularly to heart tissue, of a patient, wherein the respective tine comprises a base section connected to the header, wherein the base section is arranged between the header cap and the header insert, and wherein the respective tine comprises an anchoring section protruding out of the header wherein the anchoring section is configured to penetrate said tissue of the patient for anchoring the medical device to the tissue (e.g. to a heart wall of the patient).

According to an embodiment, the dielectric material forms a portion of the header insert, wherein said portion of the header insert contacts a face side of the flange and a bottom side of the mounting plate of the electrode, which bottom side preferably faces said face side of the flange of the housing.

According to an alternative embodiment, the dielectric material forms a portion of the header insert, wherein said portion of the header insert contacts the tines and a circumferential lateral surface of the mounting plate of the electrode.

According to yet another alternative embodiment, the dielectric material forms at least a portion of the header cap, wherein said portion of the header cap contacts the tines and the housing.

In this way, a low-pass filter can be provided between any two or more conductors of the implantable medical device, such as: between the electrode mounting plate and the housing; between the tines and the housing; between the electrode and the tines or by at least one conductor of the implantable medical device and the tissue of the patient.

According to a preferred embodiment, the respective portion (of the header insert or cap) formed by the dielectric material comprises a plastic material. Preferably, the plastic material is PEEK. In one embodiment, the plastic material is doped with a suitable conductive material, e.g. with carbon or tungsten particles, to provide a conductive part to a conductor, which forms together with the dielectric material a part of a capacitor. Such a conductor may be any conductor comprised within the header portion or the surrounding tissue.

According to an embodiment, the electrode comprises an end portion connected to the mounting plate of the electrode, which end portion protrudes out of the header, wherein a surface of said end portion forms said at least one electrode contact of the implantable medical device.

According to a further embodiment, said end portion of the electrode is surrounded by a collar (that is preferably arranged on the mounting plate), wherein the collar can comprise a steroid. Furthermore, the collar is preferably arranged flush with an outer surface of the header insert.

The medical device may be a pacemaker (see e.g. above), but can also be a cardioverter defibrillator, a neurostimulator, or a loop recorder.

In the following, embodiments of the invention as well as further features and advantages of the invention shall be explained with reference to the Figures, wherein:
- Fig. 1: shows a side view of a header of an embodiment of an implantable medical device according to the invention, where a dielectric material is integrated into the header;
- Fig. 2: shows a front view of a header of an embodiment of an implantable medical device according to the invention, wherein a dielectric material is integrated in the header and located between two electrically conductive structures of the header;
- Fig. 3: shows a front view of a header of an embodiment of an implantable medical device according to the invention, wherein here contact seals or spacer elements in the header are formed from the dielectric material;
- Fig. 4: shows a plan view of a side of a header of an embodiment of an implantable medical device according to the invention, wherein here adjusting screw seals on the header are formed from the dielectric material;
- Fig. 5: shows a plan view of a side face of a header of an embodiment of an implantable medical device according to an invention, wherein here the header comprises a region formed out of the dielectric material that is arranged between an electrical conductor and a ground plane or connects these two elements to one another;
- Fig. 6: shows a plan view of two conductors of the header, the right conductor comprising a widened portion to increase capacitance in the region of a pronounced dielectric material;
- Fig. 7: shows a cross-sectional view of an embodiment of an implantable medical device in form of an implantable leadless pacemaker, wherein the dielectric material is formed by a portion of a header insert, which portion is arranged between a flange and a bottom side of a mounting plate of an electrode of the pacemaker;
- Fig. 8: shows a cross-sectional view of a further embodiment of an implantable medical device in form of an implantable leadless pacemaker, wherein the dielectric material is formed by a portion of a header insert, which portion is arranged between the electrode and tines of the pacemaker; and
- Fig. 9: shows a cross-sectional view of a further embodiment of an implantable medical device in form of an implantable leadless pacemaker, wherein the dielectric material is formed by at least a portion of a header cap, which portion contacts the tines and the metallic housing of the pacemaker.

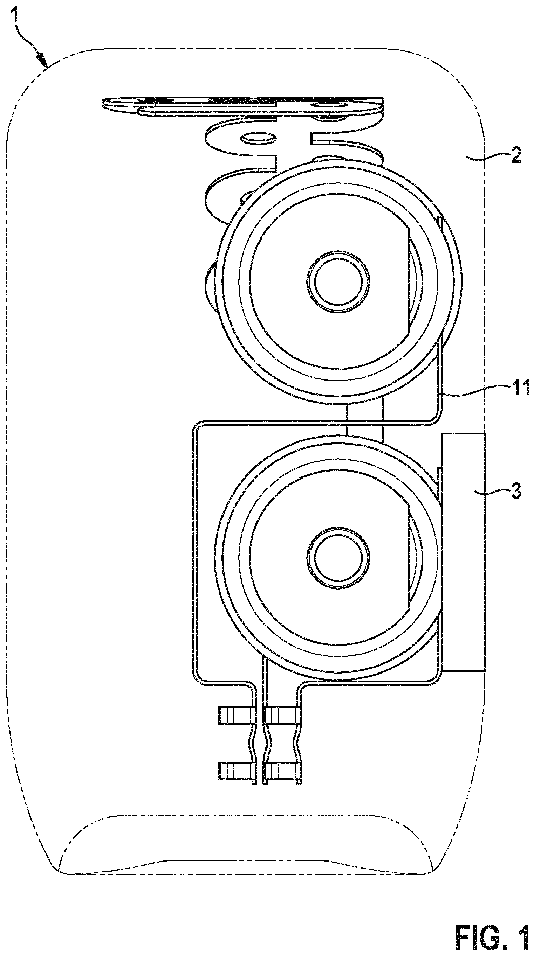

Figure 1 shows an embodiment of an implantable medical device 1 comprising a header 2 in which a suitable dielectric material 3 such as crystalline barium titanate (εᵣ = -1200) or titanium dioxide (εᵣ = -100) is integrated into the header material (typically epoxy or polysulfone (εᵣ = -3)).

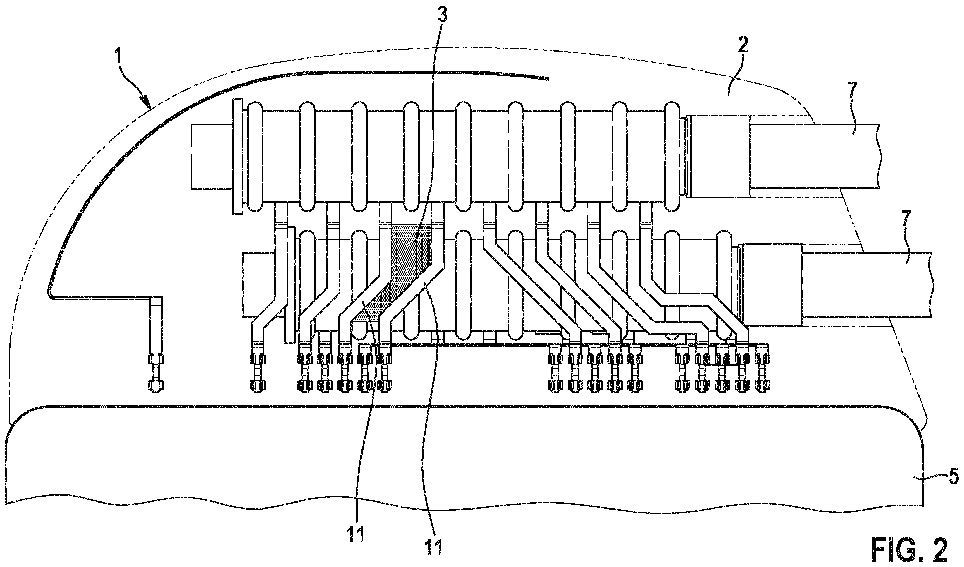

This dielectric material 3 increases the capacitance proportional to εᵣ between electrically conductive structures 11 in header 2 (for example external wiring according to Figure 2) and/or the surrounding tissue. This creates a low-pass filter that is present inside header 2. This low-pass filter diverts and distributes the RF energy applied to device 1 to reduce the likelihood of accidental stimulation due to RF rectification and the possibility of device 1 malfunction.

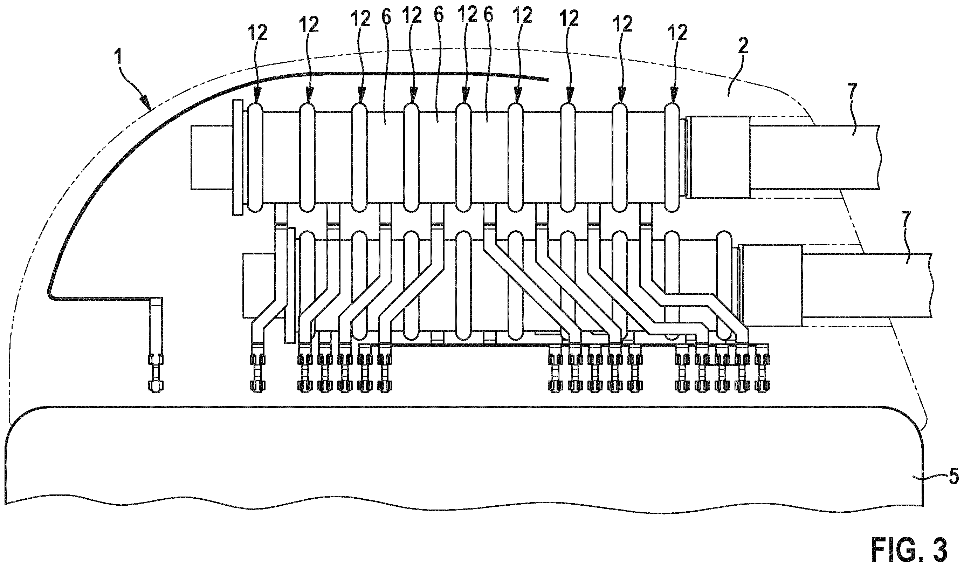

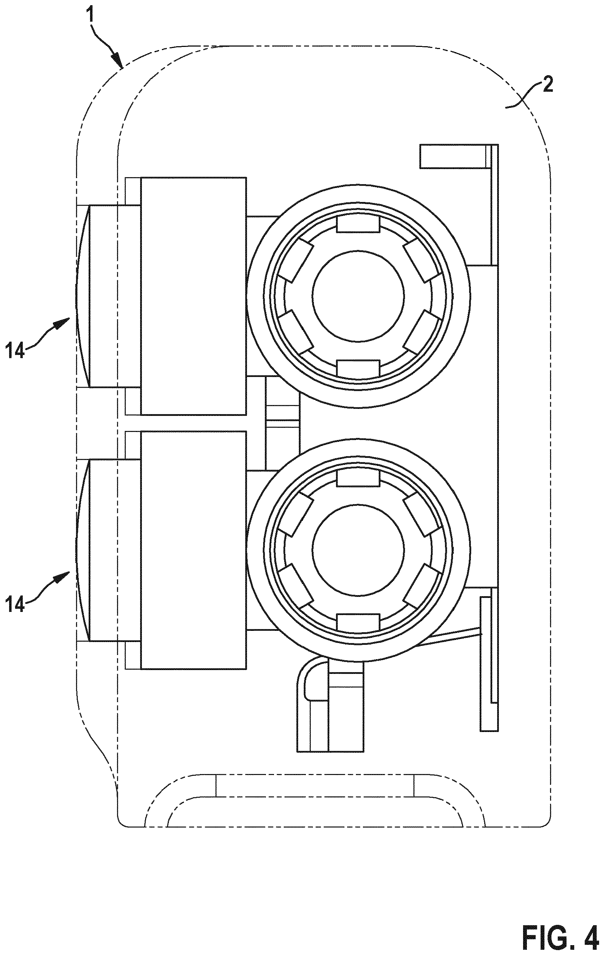

This dielectric material 3 can be placed within the entire header volume, in certain objects within the header (for example epoxy coating, preformed polysulfone sections, contact seals 12 as shown in Figure 3, spacers, adjusting screw seals 14 as shown in Figure 4) or only in certain sections of the header 2 (for example by introducing an extra impregnated polymeric object). Any of the aforementioned objects may be doped with a suitable dielectric material to increase the dielectric constant of the whole object.

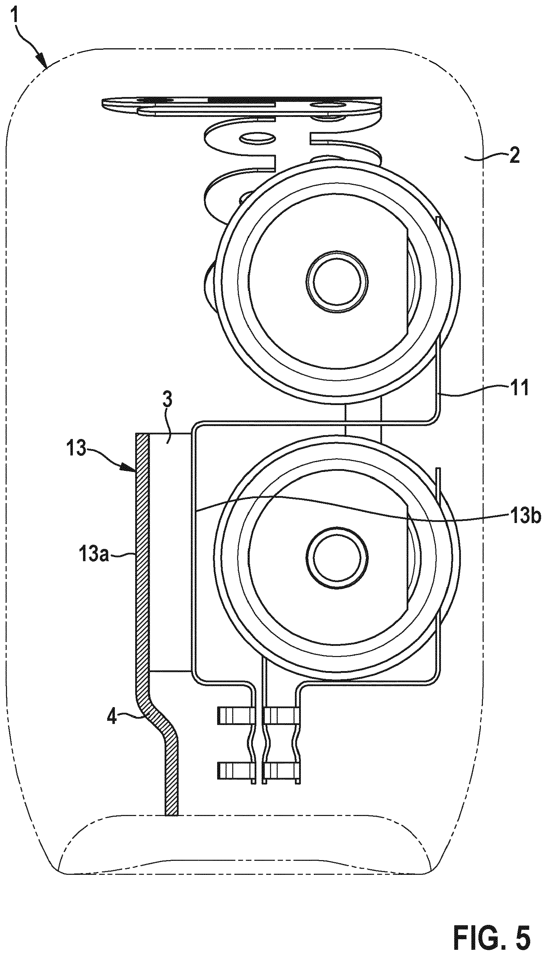

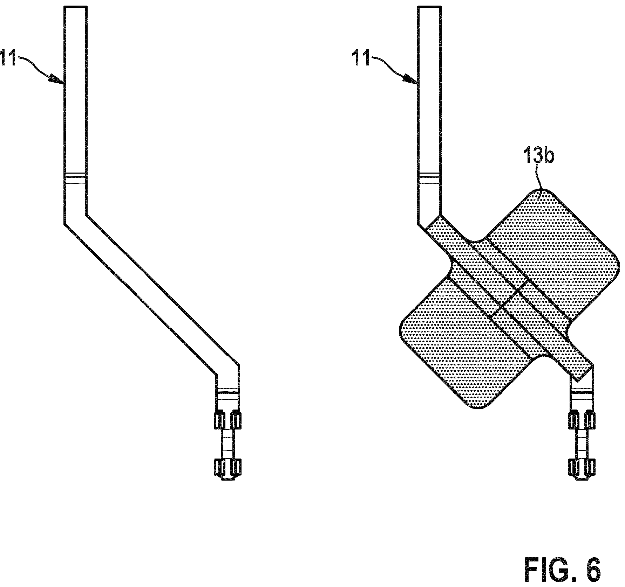

An alternative embodiment of the invention or of the device 1 is to introduce extra conductive material 4 into the header 2, which can come into contact with the device housing 5 to form, for example, a plate 13a of a capacitor 13 (see Figure 5) containing the dielectric material 3. The conductive material 4 may be a wire, a plate, or another metallic object. The other plate 13b of the capacitor can be formed by a conductor structure 11 of the header 2 (see Figure 6 below).

In some embodiments, this extra conductive material could also be doped into the header insulation material to provide a conductive path to tissue, housing 5, or between the header contacts 6 used to contact an electrode lead 7 of device 1. Examples of weakly conductive materials suitable for this purpose are tungsten powder or carbon. For example, the header insulating material may comprise up to 40% by weight barium sulfate, up to 40% by weight barium subcarbonate, or up to 40% by weight bismuth oxychloride in order to provide a dielectric property; or up to 80% by weight tungsten to provide the conductive path to the tissue. Alternatively, the header insulating material may comprise conductive carbon-infused polymers.

For the embodiment shown in Figure 5, the outer wiring or conductor structure 11 can be widened inside header 2 to form a plate 13b as shown in Figure 6 on the right hand side. Plate 13b or the outer wiring 11 can assume any number of shapes, such as rectangular, circular, triangular, etc., to optimize placement within the header 2. Additional embodiments may include openings in the plate 13b or in the outer wiring 11 to strategically allow capacitive coupling between certain objects (and especially to avoid capacitive coupling between other objects).

According to a further embodiment, the low-pass filter described above can also be used in an active implantable medical device (AIMD), such as an intracardiac pacemaker (leadless pacer) or in a loop recorder, to filter radiofrequency energy incident on the device.

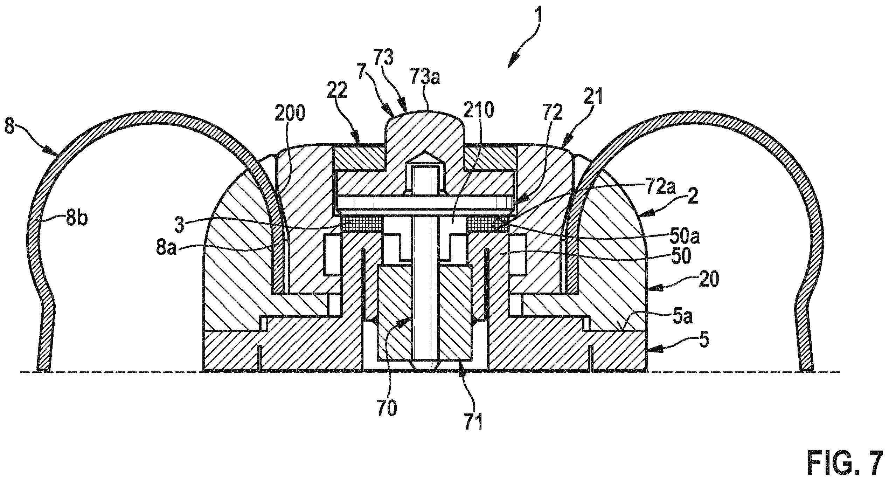

According to the further embodiments shown in Figs. 7 to 9, the implantable medical device 1 can be an implantable leadless pacemaker 1 for applying electrical pacing pulses to a heart of a patient. Such a pacemaker 1 is an intracardiac pacemaker that does not comprise a flexible electrode lead but an electrode 7 having an electrode contact 73a that is formed on the header portion 2 of the implantable medical device 1.

Such a pacemaker 1 can comprise a metallic housing 5 that can comprise an elongated cylindrical shape, wherein the housing 5 encloses a battery and an electrical circuit (not shown in Figs. 7 to 9) of the pacemaker 1 that is configured to generate the pacing pulses to be applied to tissue of the patient via the electrode 7. In Figs. 7 to 9 only a top side 5a of the housing 5 with a metallic flange 50 welded to the housing 5 is shown, wherein an electrically insulating body 71 (for example out of a ceramic material) is connected, particularly brazed, to the metallic flange 50 to form a feedthrough. A metallic pin 70 extends through and is connected, particularly brazed with, this body 71 and is electrically connected to a metallic mounting plate 72 of the electrode 7.

The pacemaker 1 can be configured to be implanted into a ventricle of the heart of a patient. Preferably, the pacemaker 1 comprises tines 8 connected to the header 2 to anchor the pacemaker 1 to the ventricle. In this anchored state, an end portion 73 of the electrode 7, that protrudes out of the header portion 2, contacts the heart tissue of the patient via a surface 73a of said end portion 73, which surface 73a thus forms an electrode contact 73a. Furthermore, said end portion 73 of the electrode 7 can be surrounded by a collar 22 comprising a steroid, wherein particularly the collar 22 is arranged flush with an outer surface of the header insert 21.

The flange 50 and/or the housing 5 can be formed out of a titanium alloy. Further, the pin 70 and/or the mounting plate 72 can be formed out of a metal comprising platinum and/or iridium. Furthermore, the electrode 7, particularly its end portion 73 and the electrode contact 73a can also be formed out of a metal comprising platinum and/or iridium.

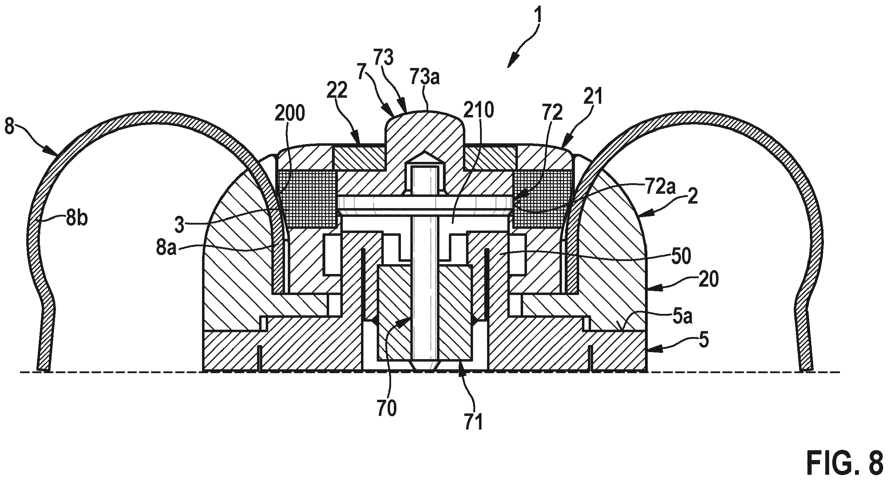

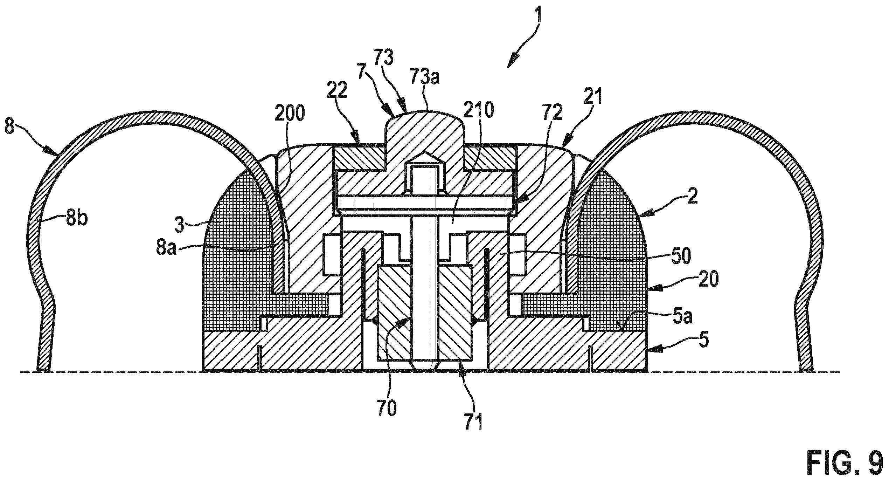

In case of such an implantable leadless pacemaker 1 the dielectric material 3, as part of a capacitor 13 in order to provide a low-pass filter for e.g. the electrode 73 or the housing 5 of the pacemaker, can be placed between any two or more conductors of the implant 1, such as: between the electrode mounting plate 72 and the housing 5 (cf. Fig. 7); between the tines 8 and the housing 5 (cf. Fig. 9); between the electrode 7 and the tines 8 (cf. Fig. 8). In some cases, the surrounding tissue may be used as a weak conductor to form "a conductive plate" of above mentioned capacitor.

The dielectric material 3 can be formed for example by doping a portion of the insulating material between the conductors, up to entire objects. Example components of the pacemaker 1 include the header cap 20, the header insert 21, and the steroid collar 22.

Specifically, as shown in Fig. 7, the header 2 of the pacemaker 1 may comprise a circumferential header cap 20 and a header insert 21 arranged in a through opening 200 of the header cap 20, wherein the header insert 21 also comprises a through opening 210 through which an electrically conducting pin 70 extends that is electrically conductively connected to the electrode 7 via a mounting plate 72 of the electrode 7. Particularly, the pin 70 extends through the body 71 that is connected to the circumferential metallic flange 50, wherein the metallic flange is fixed, particularly welded, to the housing 5. Flange 50, body 71 and pin 70 form a hermetically sealed feedthrough to connect the battery and electrical circuit housed in housing 5 to the electrode 7. The electrode 7 is connected to the header 2 via electrically conducting pin 70.

As shown in Fig. 7, the implantable medical device 1 comprises tines 8 for anchoring the implantable medical device 1 to tissue of the patient, wherein the respective tine 8 comprises a base section 8a connected to the header portion 2, wherein the base section 8a is arranged between the header cap 20 and the header insert 21. Further, the respective tine 8 comprises an anchoring section 8b protruding out of the header portion 2, wherein the anchoring section 8b is configured to penetrate said tissue of the patient for anchoring the pacemaker 1.

In the embodiment of the implantable medical device 1 shown in Fig. 7, the dielectric material 3 forms a portion 3, preferably an annular portion 3, of the header insert 21, wherein said portion 3 of the header insert 21 contacts a face side 50a of the flange 50 and a bottom side 72a of the mounting plate 72 of the electrode 7, which bottom side 72a faces said face side 50a of the flange 50.

In contrast to Fig 7, Fig. 8 shows an embodiment where the dielectric material 3 forms a portion 3 of the header insert 21 that contacts the base sections 8a of the tines 8 and a circumferential lateral surface 72a of a mounting plate 72 of the electrode 7. Also here, the portion 3 can be an annular portion 3.

Furthermore, according to yet another embodiment shown in Fig. 9, the dielectric material 3 forms at least a portion 3 of the header cap 20, wherein said portion 3 of the header cap 20 contacts the base sections 8a of the tines 8, the flange 50 and the housing 5, particularly the top side 5a of the housing 5, to which said flange 50 is fixed, particularly welded.

In the embodiments shown in Figs. 7 to 9, the header cap 20 and/or the header insert 21 can comprise a plastic material such as PEEK. Preferably, the respective portion/dielectric material 3 is formed by doping the corresponding region of the plastic material (for example PEEK) with suitable dielectric materials such as BaSO₄, BaTiO3. Optionally, one or more conductive paths, e.g. to the tissue may be formed within the header cap 20 and/or header insert 21, by carbon or tungsten particles (or other suitable particles).

The present invention can be applied with advantage to all conceivable device types (neuro, heart, monitor, etc.). The invention has very little effect on, for example, the design of the headers, as the dielectric material and/or conductive material can be doped in small amounts into the epoxy or polysulfone headers or other insulating header objects. The invention reduces the amount of induced currents entering the device so that a larger number of cable types and lengths can be MRI-conditional.

The invention can also be combined with leads without MRI capability to increase MRI capability. Further, the header design can modify the coupling between the RF input points, reducing the hardware's EMC protection effort.

Furthermore, particularly in case of implantable leadless pacemakers, the invention allows including electromagnetic interference (EMI) protection without adding further components, which is advantageous due to the fact that leadless pacemaker schematics have strict component count and size limits.

Furthermore, the invention allows using the leadless pacemaker fixation tines as additional shielding at high frequency by capacitively coupling them to the housing. The leadless pacemaker design may be configured to keep the tines floating relative to the housing for requirements related to the therapeutic operation. This solution preserves that the tines are floating at low frequency while adding the advantage that they are shunted to the housing at high frequency.

## Claims

1. An implantable medical device (1), comprising:
- a header portion (2) attached to a housing (5) of the device (1), and
- at least one electrode (7) which is electrically conductively connected or connectable to the header portion (2), or which is arranged at or extends through the header portion (2), wherein the at least one electrode (7) comprises at least one electrode contact (73a) for contacting tissue of the patient and for applying electrical stimulation pulses to the tissue and/or detecting electrical pulses from the tissue.
**characterized in that**
the header (2) comprises a dielectric material (3) that is configured to capacitively couple RF energy acting on the device (1) and/or on the at least one electrode (7) to a conductor (5) of the implantable medical device (1) or to the tissue of the patient when the tissue touches the header (2) of the device (1).

2. The implantable medical device according to claim 1, **characterized in that** that the conductor is the housing (5).

3. The implantable medical device according to claim 1 or 2, **characterized in that** the at least one electrode (7) is an electrode lead (7).

4. The implantable medical device according to one of the preceding claims, **characterized in that** the dielectric material (3) is one of or comprises one of: crystalline barium titanate, titanium dioxide, barium sulfate, barium subcarbonate, bismuth oxychloride.

5. The implantable medical device according to one of the preceding claims, **characterized in that** the dielectric material (3) is arranged between two electrically conductive structures (8, 11, 13) of the header portion (2).

6. The implantable medical device according to one of the preceding claims, **characterized in that** the dielectric material (3) is comprised by at least one of: a thermoset or thermoplastic coating or header housing, particularly an epoxy coating, a preformed thermoplastic section, e.g. made of polysulfone, a contact seal (12), a spacer, or an adjusting screw seal (13)

7. The implantable medical device according to one of the preceding claims, **characterized in that** the header portion (2) comprises at least one conductor (11, 13), which forms together with the dielectric material (3) a part of a capacitor.

8. The implantable medical device according to claim 1, **characterized in that** the header portion (2) comprises a circumferential header cap (20) and a header insert (21) arranged in a through opening (200) of the header cap (20), and wherein the header insert (21) comprises a through opening (210) through which an electrically conducting pin (70) extends that is electrically conductively connected to the electrode (7), or the electrically conducting pin forms the at least one electrode (7), wherein the pin (70) extends through an electrically insulating body (71) that is connected to a circumferential metallic flange (50) of the housing (5).

9. The implantable medical device according to claim 8, **characterized in that** the implantable medical device (1) comprises a plurality of tines (8) for anchoring the implantable medical device (1) to tissue of a patient, wherein the respective tine (8) comprises a base section (8a) connected to the header portion (2), wherein the base section (8a) is arranged between the header cap (20) and the header insert (21), and wherein the respective tine (8) comprises an anchoring section (8b) protruding out of the header (2), wherein the anchoring section (8b) is configured to penetrate said tissue of the patient.

10. The implantable medical device according to claim 8 or 9, **characterized in that** the dielectric material (3) forms a portion (3) of the header insert (21), wherein said portion (3) of the header insert (21) contacts a face side (50a) of the flange (50) and a bottom side (72a) of a mounting plate (72) of the at least one electrode (7).

11. The implantable medical device according to claim 9, **characterized in that** dielectric material (3) forms a portion (3) of the header insert (21), wherein said portion (3) of the header insert (21) contacts the base sections (8a) of the tines (8) and a circumferential lateral surface (72a) of a mounting plate (72) of the at least one electrode (7).

12. The implantable medical device according to claim 9, **characterized in that** dielectric material (3) forms at least a portion (3) of the header cap (20), wherein said portion (3) of the header cap (20) contacts the base sections (8a) of the tines (8) and the housing (5).

13. The implantable medical device according to one of the claims 10 to 12, **characterized in that** said portion (3) formed by the dielectric material (3) comprises a plastic material, wherein preferably said plastic material is PEEK, and wherein the plastic material optionally comprise a conductive region, formed by doping the conductive region with a suitable conductive material, such as carbon or tungsten.

14. The implantable medical device according to one of the claims 8 to 13, **characterized in that** the at least one electrode (7) comprises an end portion (73) protruding out of the header (2), wherein a surface (73a) of said end portion (73) forms said electrode contact (73a)

15. The implantable medical device according to one of the claims 8 to 14, **characterized in that** the implantable medical device (1) is an implantable leadless pacemaker.
